# EUROPEAN PATENT SPECIFICATION

(11) **EP 1 347 751 B1**
(45) Date of publication and mention of the grant of the patent: **28.04.2010**
(21) Application number: 01959595.8
(22) Date of filing: 06.08.2001
(51) Int. Cl.: A61K 31/4025, A61P 35/00

(54) **METHODS OF TREATING BONE CANCER AND THE PAIN ASSOCIATED THEREWITH USING ENDOTHELIN ANTAGONISTS**
VERWENDUNG VON ENDOTHELIN ANTAGONISTEN ZUR BEHANDLUNG VON KNOCHENKREBS UND DAMIT VERBUNDENEN SCHMERZEN
PROCEDE POUR TRAITER LE CANCER ET LA DOULEUR PROVOQUEE PAR LE CANCER AU MOYEN D'ANTAGONISTES D'ENDOTHELINE

(30) Priority: 07.08.2000 US 633389
(43) Date of publication of application: 01.10.2003
(73) Proprietor: ABBOTT LABORATORIES, Abbott Park IL 60064-3500 (US)
(72) Inventor: JANUS, Todd, J., Gurnee, IL 60031 (US); PADLEY, Robert, J., Lake Bluff, IL 60044 (US)
(74) Representative: Modiano, Micaela Nadia
(86) International application number: PCT/US2001/024716
(87) International publication number: WO 2002/011713

(56) References cited:
- WO-A-00/36918
- WO-A-00/36918
- WO-A-01/00198
- WO-A-01/00198
- WO-A-97/30045
- WO-A-97/30045
- WO-A-99/06397
- WO-A-99/06397
- NELSON J B ET AL: "NEW BONE FORMATION IN AN OSTEOBLASTIC TUMOR MODEL IS INCREASED BY ENDOTHELIN-1 OVEREXPRESSION AND DECREASED BY ENDOTHELIN A RECEPTOR BLOCKADE" UROLOGY, BELLE MEAD, NJ, US, vol. 53, no. 5, May 1999 (1999-05), pages 1063-1069, XP001037666 ISSN: 0090-4295
- YIN J J ET AL: "Osteoblastic bone metastases: Tumor-produced endothelin-1 mediates new bone formation via the endothelin A receptor." CANCER, vol. 88, no. 12, 15 June 2000 (2000-06-15), pages 3093-3094, XP008009105 Second North American Symposium on Skeletal Complications of Malignancy;Montreal, Canada; October 15-16, 1999 ISSN: 0008-543X
- LAHAV R ET AL: "AN ENDOTHELIN RECEPTOR B ANTAGONIST INHIBITS GROWTH AND INDUCES CELL DEATH IN HUMAN MELANOMA CELLS IN VITRO AND IN VIVO" PROCEEDINGS OF THE NATIONAL ACADEMY OF SCIENCES OF USA, NATIONAL ACADEMY OF SCIENCE. WASHINGTON, US, vol. 96, no. 20, September 1999 (1999-09), pages 11496-11500, XP001026099 ISSN: 0027-8424
- HIRUMA Y ET AL: "ENDOTHELINS INHIBIT THE MINERALIZATION OF OSTEOBLASTIC MC3T3-E1 CELLS THROUGH THE A-TYPE ENDOTHELIN RECEPTOR" AMERICAN JOURNAL OF PHYSIOLOGY, AMERICAN PHYSIOLOGICAL SOCIETY, BETHESDA, MD, US, vol. 44, no. 4, PART 2, October 1998 (1998-10), pages R1099-R1105, XP008009111 ISSN: 0002-9513
- NELSON J B ET AL: "NEW BONE FORMATION IN AN OSTEOBLASTIC TUMOR MODEL IS INCREASED BY ENDOTHELIN-1 OVEREXPRESSION AND DECREASED BY ENDOTHELIN A RECEPTOR BLOCKADE" UROLOGY, BELLE MEAD, NJ, US, vol. 53, no. 5, May 1999 (1999-05), pages 1063-1069, XP001037666 ISSN: 0090-4295
- YIN J J ET AL: "Osteoblastic bone metastases: Tumor-produced endothelin-1 mediates new bone formation via the endothelin A receptor." CANCER, vol. 88, no. 12, 15 June 2000 (2000-06-15), pages 3093-3094, XP008009105 Second North American Symposium on Skeletal Complications of Malignancy;Montreal, Canada; October 15-16, 1999 ISSN: 0008-543X
- LAHAV R ET AL: "AN ENDOTHELIN RECEPTOR B ANTAGONIST INHIBITS GROWTH AND INDUCES CELL DEATH IN HUMAN MELANOMA CELLS IN VITRO AND IN VIVO" PROCEEDINGS OF THE NATIONAL ACADEMY OF SCIENCES OF USA, NATIONAL ACADEMY OF SCIENCE. WASHINGTON, US, vol. 96, no. 20, September 1999 (1999-09), pages 11496-11500, XP001026099 ISSN: 0027-8424

## Description

### Field of the Invention

The invention is directed to an endothelin ET-A receptor antagonist having formula III for inhibiting bone metastases in prostate cancer patients.

### Background of the Invention

Endothelin (ET), a 21 amino acid peptide, is produced by enzymatic cleavage of a precursor peptide by an endothelin converting enzyme. First discovered in vascular endothelial cells, ET and ET/ET receptor binding are now known to modulate smooth muscle tone, blood flow, cell proliferation and differentation, protein synthesis, and metabolic function in a variety of tissues and cell types such as ovary, prostate, skin, and brain.

ET/ET receptor binding has been shown to constrict arteries and veins; increase mean arterial blood pressure; decrease incardiac output; increase cardiac contractility in vitro; stimulate mitogenesis in vascular smooth muscle cells in vitro; contract non-vascular smooth muscle such as guinea pig trachea, human urinary bladder strips and rat uterus in vitro; increase airway resistance in vivo; induce formation of gastric ulcers; stimulate release of atrial natriuretic factor in vitro and in vivo; increase plasma levels of vasopressin, aldosterone, and catecholamines; inhibit release of renin in vitro; and stimulate release of gonadotropins in vitro.

ET/ET receptor binding also causes vasoconstriction on vascular smooth muscle (Nature 332 411 (1988), FEBS Letters 231 440 (1988) and Biochem. Biophys. Res. Commun. 154 868 (1988)). In fact, an anti-ET antibody has been shown to ameliorate adverse effects of renal ischemia on renal vascular resistance and glomerular filtration rate (J. Clin. Invest. 83 1762 (1989)). In addition, an anti-ET antibody attenuated both the nephrotoxic effects of intravenously administered cyclosporin (Kidney Int. 37 1487 (1990)) and the infarct size in a coronary artery ligation-induced myocardial infarction model (Nature 344 114 (1990)).

A nonpeptide ET antagonist prevents post-ischaemic renal vasoconstriction in rats, prevents the decrease in cerebral blood flow due to subarachnoid hemorrhage in rats, and decreases MAP in sodium-depleted squirrel monkeys when dosed orally (Nature 365: 759-761 (1993)). A similar effect of an ET antagonist on arterial calibera has also been recently reported (Biochem. Biophys. Res. Comm., 195: 969-75 (1993).

An ET receptor antagonist reduced injury in a rat model of colitis (EUR. J. Pharmacol. 1996, 309, 261-269) and prevented ischemia-reperfusion injury in kidney transplantation (Transplant Int 1996, 9, 201-207). The use of ET antagonists in the treatment of angina, pulmonary hypertension, Raynaud's disease, and migraine has also been suggested (Drugs 1996, 51,12-27). In malignant growth disorders, ET and its growth-promoting effects have been best characterized in prostate cancer, (Nature Medicine 1995, 1, 944-949) wherein ET acts as a modulator in osteoblastic bone lesion (UROLOGY 53:1063-1069, 1999).

Given the results from these and other reports which illuminate the role of ET/ET receptor binding in disease states, and the knowledge that blocking ET/ET receptor binding results in improvement or reversal of endothelin-induced disease states, agents which antagonize ET/ET receptor binding activity, designated as ET receptor antagonists, can provide substantial benefit in many therapeutic areas.

Urology 53 (5) 1063-1069 (1999) reports a study finding that new bone formation in an osteoblastic tumor model is decreased by endothelin A receptor blockage. The tumor cell line WISH was used in the study. The authors recognized that their findings may not be directly applicable to human prostate cancer. Cancer Supplement, 88 (12) 3093-3094 (June 15, 2000) states, in connection with plasma ET-1 concentrations of ET-1 in men with advanced prostate carcinoma, that a causal role for ET-1 in osteoblastic metastases has not been demonstrated.

### Summary of the Invention

In accordance with the present invention, there is disclosed a compound of formula III: 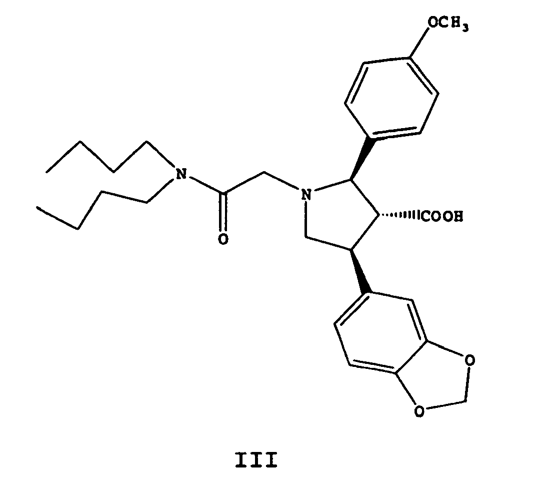 or a pharmaceutically acceptable salt thereof for inhibiting bone metastases in a man having prostate cancer by oral administration of a therapeutically effective amount to the man in need thereof.

A compound of formula III is also known as ABT-627. According to the invention, such compound is useful for preventing new bone metastases in a prostate cancer patient.

The compound of formula III can be administered orally in therapeutically acceptable formulations, optionally in the presence of a co-therapeutic agent, for inhibiting bone metastases in a man having prostate cancer.

### Brief Description of the Drawings

Figure 1 illustrates levels of interleukin-6 (IL-6) in a subject population treated with a placebo or 2.5 mg or 10 mg ABT-627.
Figure 2 illustrates levels of prostate specific antigen (PSA) in a subject population treated with a placebo or 2.5 mg or 10 mg of ABT-627.
Figure 3 illustrates VAS score levels relating to pain assessment in a subject population treated with a placebo or 2.5 mg or 10 mg of ABT-627.
Figure 4 illustrates crosslinked N-telopeptides (degradation) in a subject population treated with a placebo or 10 mg ABT-627.
Figure 5 illustrates bone alkaline phosphatase (BAP)(formation) in a subject population treated with a placebo or 10 mg ABT-627.
Figure 6 illustrates skeletal involvement in a subject population treated with a placebo or 10 mg ABT-627.
Figure 7 illustrates acid phosphatase levels in a subject population treated with a placebo or 10 mg ABT-627.

### Detailed Description of the Invention

An endothelin receptor antagonist having formula III is employed in the practice of the present invention. Endothelins are a family of peptides mainly synthesized and released by endothelial cells. The term "endothelin" refers to a family of homologous 21-amino acid peptides found in 3 distinct isoforms: ET-1, ET-2, and ET-3.

The term "endothelin ET-A receptor antagonist" includes both compounds which antagonize the ET-A receptor in a selective manner, as well as compounds which antagonize the ET-A receptor in a non-selective manner. An example of the latter type of compound would be a compound that antagonizes the ET-A receptor and also antagonizes the ET-B receptor.

The term "primary cancer" means cancer in a specific tissue, which is first in time or in order of development. Primary cancers include, but are not limited to, breast, prostate, lung, kidney, thyroid, brain, heart, intestine, ovary, myeloma, lymphoma, sarcoma, and osteosarcoma.

The term "cancer-related pain" includes pain which arises from direct invasion or expansion of a tumor into tissue, such as bone or nerve; pain which arises from the consequences of tumor invasion or expansion, such as bone collapse due to cancer erosion or secretion of noxious agents which modulate or produce pain; and pain mediated by ischemia, i.e. reduced blood flow.

A compound of the above formula III may be synthesized by methods provided in WO 99/06397.

The term "inhibit" is defined to include its generally accepted meaning which includes preventing, prohibiting, restraining, and slowing, stopping or reversing progression, or severity, and holding in check and/or treating existing characteristics. The use disclosed herein includes both medical therapeutic and/or prophylactic treatment, as appropriate.

The compound of formula III can be used in the form of salts derived from inorganic or organic acids. These salts include the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid.

Basic addition salts can be prepared *in situ* during the final isolation and purification of the compounds of formula I, or separately by reacting the carboxylic acid function with a suitable base such as the hydroxide, carbonate or bicarbonate of a pharmaceutically acceptable metal cation or with ammonia, or an organic primary, secondary or tertiary amine. Such pharmaceutically acceptable salts include, cations based on the alkali and alkaline earth metals, such as sodium, lithium, potassium, calcium, magnesium, aluminum salts, as well as nontoxic ammonium, quaternary ammonium, and amine cations, including ammonium, tetramethylammonium, tetraethylammonium, methylamine, dimethylamine, trimethylamine, triethylamine, ethylamine. Other representative organic amines useful for the formation of base addition salts include diethylamine, ethylenediamine, ethanolamine, diethanolamine, piperazine.

The compound of formula III is useful for antagonizing endothelin in humans or other mammals. Total daily dose administered to a host in single or divided doses may be in amounts, for example, from 0.001 to 1000 mg/kg body weight daily and more usually 0.1 to 100 mg/kg for oral administration or 0.01 to 10 mg/kg for parenteral administration. Dosage unit compositions may contain such amounts of submultiples thereof to make up the daily dose.

Pharmaceutical formulations may be prepared by procedures known in the art. The amount of active ingredient that may be combined with the carrier materials to produce a single dosage form will vary depending upon the host treated and the particular mode of administration.

It will be understood, however, that the specific oral dose level for any particular prostate cancer patient will depend upon a variety of factors including the activity of the specific compound employed, the age, body weight, general health, diet, time of administration, rate of excretion, drug combination, and the severity of the particular disease undergoing therapy.

The compound of formula III may be administered orally in dosage unit formulations containing conventional nontoxic pharmaceutically acceptable carriers, adjuvants, and vehicles as desired.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, and sweetening, flavoring, and perfuming agents.

The compound of formula III can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients. The preferred lipids are the phospholipids and phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq.

A representative solid dosage form, for example, a tablet or a capsule, comprises:

| | |
|---|---|
| Compound of the invention: | 35% w/w |
| Starch, Pregelatinized, NF | 50% w/w |
| Microcrystalline Cellulose, NF | 10% w/w |
| Talc, Powder, USP | 5% w/w |

While the compound of formula III can be administered as the sole active therapeutic agent, it can also be used in combination with one or more co-therapeutic agents, such as anticancer drugs or treatments including, hormonal agents, such as leuprolide (Lupron^{®}); gonadorelin antagonists, such as goserelin (Zoladex^{®}) and abarelix; bicalutamide; nilutamide; flutamide; vitamin D; vitamin D analogues; estrogen and estrogen analogues, such as diethylstibestrol; prednisone; hydrocortisone; ketoconazole; cyproterone acetate; progesterone; 5-alpha reductase inhibitors, such as finasteride; bone-seeking radionuclides, such as samarium (Quadramet^{®}), strontium (Metastron^{®}), and ¹⁸⁶ rhenium; external beam radiation, including three dimensional conformal radiation; brachytherapy, which is the implantation of radioactive seeds directly into the prostate; monoclonal antibodies such as trastuzumab (Herceptin^{®}); anti-angiogenic agents such as thrombospondin peptide or kringle 5; matrix metalloproteinase inhibitors; farnesyl transferase inhibitors; lycopenes; urokinase; plasminogen activator inhibitors; plasminogen activator receptor blockers; apoptosis inducers; selective and non-selective alpha blockers; platinum agents, such as cis-platinum and carbo-platinum; taxane class agents, such as docitaxil and paclitaxil; estramustine; gemcytabine; adriamycin; doxorubicin; daunorubicin; mitoxantrone; vinblastine; vincristine; capecitabine; irinotecan; topotecan; 5-fluorouracil; interferons; cytoxan; methotrexate; cytokines, such as IL-2; PPAR agonists, such as thiazolidine diones; retinoid-type agents, 5-lipooxygenase inhibitors, such as zyfo (Zilueton^{®}), COX-2 inhibitors; gene-therapy based therapeutics, including sense and anti-sense genes; cholesterol lowering drugs, such as lovastatin, pravastatin, and simvistatin; bisphosphonates; osteoprotegrin; and antibodies, both monoclonal and polyclonal; antibody-coupled radionucleotides; antibody-coupled cytotoxic agents; antibody-coupled radionucleotides; viral-vector delivered agents; vaccines directed at protein, carbohydrate, or nucleic acid targets; aminoglutethimide; and suramin.

These combinations can be administered as separate compositions or as a single dosage form containing both or all agents. When administered as a combination, the therapeutic agents can be formulated as separate compositions, which are given at the same time or different times, or the therapeutic agents can be given as a single composition.

In addition, the compound of formula III can be used in combination with one or more co-therapeutic agents which impede net bone loss, such as estrogens, bisphosphonates, and estrogen receptor modulators, such as raloxifene, and calcitonin.

The compound of formula III can additionally be administered in combination with surgery, such as radical prostatectomy, cryotherapy, transurethral resection of the prostate as an adjuvant, or prior to surgery as a neoadjuvant agent.

The following examples relate to different aspects of the treatment of men with prostate cancer using a compound of formula III. The present invention relates to a compound of formula III for inhibiting bone metastases in a man having prostate cancer as defined in the appended claims.

### Examples

Studies were performed on male subjects with asymptomatic hormone refractory prostate cancer with rising PSA levels and on male subjects with symptomatic hormone refractory prostate cancer with rising PSA levels and pain. Subjects in the phase II studies had castrate levels of testosterone, either due to pharmacologic intervention, via leuprolide (Lupron^{®}) or goserelin (Zoladex^{®}), or via surgical castration. Subjects received ABT-627 or placebo. The following tests were conducted:
ABT-627 was formulated in 2.5 and 10 mg doses. An oral liquid formulation of ABT-627 was also prepared as follows: 1 mg/ml ABT-627, 50% glycerin, 14% alcohol, and water. Matching placebos were also provided.

A number of recognized or putative biochemical markers of disease progression have been used to monitor treatment of individuals with prostate cancer. Among these markers are serum Prostate Specific Antigen (PSA), serum acid Phosphatase, Interleukin-6, and Chromagranin-A. As currently accepted, favorable treatment is marked by a decrease or slower rate of increase for PSA, acid phosphatase, and Interleukin-6, while a favorable response is marked by an increase in Chromagranin-A.

Serum samples were obtained from subjects during treatment with the ET antagonist ABT-627 in order to determine PSA, acid phosphatase, IL-6, and Chromagranin-A values.

### Prostate Specific Antigen Level Assay (Reference example)

The effect of ABT-627 administration on prostate specific antigen (PSA) levels in human subject serum samples was determined using the procedure described in the Chiron Diagnostics ACS: Centaur PSA2 Assay. This assay is a two-site sandwich immunoassay which uses direct chemiluminescense and constant amounts of two antibodies. The first antibody, the Lite Reagent, is an affinity purified polyclonal sheep anti-PSA antibody labeled with acridinium ester. The Lite Reagent is purchased as a 5.0 mL reagent pack comprising the polyclonal sheep anti-PSA antibody (3.1 µg) in buffered saline with sodium azide (0.1%). The second antibody, the Solid Phase, is a monoclonal mouse anti-PSA antibody covalently coupled to paramagnetic particles. The Solid Phase is purchased as a 25.0 mL reagent pack comprising the covalently coupled monoclonal mouse anti-PSA antibody (316 µg) in buffered saline with sodium azide (0.1%). The assay was performed at Quintiles Laboratories (Smyrna, GA) using Chiron Diagnostics ACS: Centaur^{®} Automated Chemiluminescence Systems.

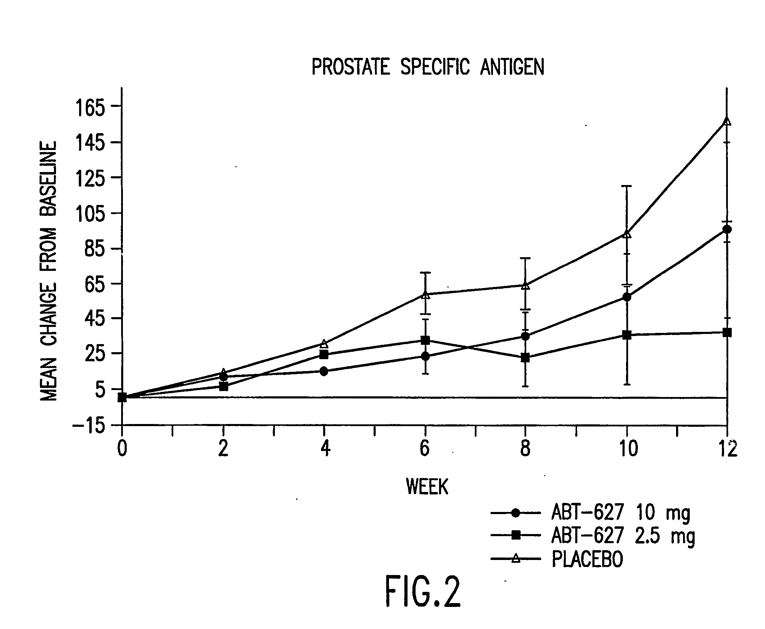

Briefly, a subject population was treated with a placebo or 2.5 mg or 10 mg of ABT-627. Blood samples were collected, allowed to adequately clot, centrifuged at 1000 x g for 15-20 minutes, and stored at -20 °C if not assayed within 48 hours. A cuvette was charged sequentially with serum, Lite Reagent (50 µL), and Solid Phase (250 µL). The resulting mixture was incubated for 7.5 minutes at 37 °C, separated, and treated with the solution of Acid Reagent and Base Reagent to initiate the chemiluminescent reaction. A direct relationship exists between the amount of PSA present in the patient sample and the RLU's (relative light units) detected. As shown by the area under the curve (AUC) in Figure 2, the rate of increase of PSA in the serum samples decreases after the administration of ABT-627, demonstrating the effectivness of ABT-627 as an agent for treating prostate cancer.

### Acid Phosphatase Levels (Reference example)

The effect of ABT-627 administration on Acid Phosphatase levels in human subject serum samples was determined at Quintiles Laboratories using the chemical test described in Sigma Diagnostics Acid Phosphatase (ACP) Procedure No. 435. The enzyme Acid Phosphatase (ACP) catalyzes the hydrolysis of alpha-naphthyl phosphate to alpha-naphthol and inorganic phosphate. The alpha-naphthol immediately reacts with fast red TR salt to produce a yellow chromophore with an absorbance maximum at 405 nm. The rate of increase in absorbance at 405 nm is directly proportional to ACP activity in the sample. ACP activity was determined in the presence and absence of L-tartrate, the difference being attributed to prostatic acid phosphatase activity.
Briefly, a subject population was treated with a placebo or 2.5 mg or 10 mg of ABT-627. Blood samples were collected, allowed to adequately clot, centrifuged at 1000 x g for 15-20 minutes, and stored at -20 °C if not assayed within 48 hours. Assays were performed on a Hitachi Spectrophotomer. A cuvette was charged sequentially with ACP reagent (1 mL), prepared as described in the assay protocol, and serum (0.1 mL). The mixture was agitated and incubated for 5 minutes, and an absorbance (A) at 405 nm (versus water as a reference) was read to provide an initial absorbance. The mixture was incubated for another 5 minutes, and a second absorbance was read to provide a final absorbance. A change A/5 minute value was obtained by subtracting the initial absorbance from the final absorbance and was used to calculate total ACP activity.

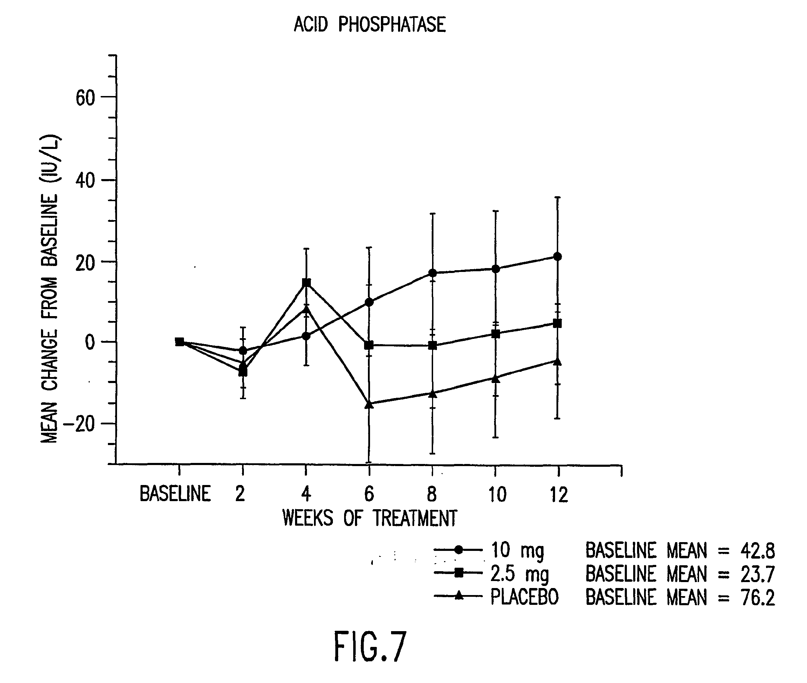

To provide the tartrate-resistant acid phosphatase activity, the above procedure was repeated with the addition of ACP tartrate reagent (0.01 mL) to the cuvette containing the ACP reagent and mixing before adding the serum. Prostatic acid phosphatase activity was calculated by subtracting the the tartrate-resistant acid phosphatase activity from the ACP activity. As shown shown by the (AUC) in Figure 7, the rate of increase and the average change from baseline for acid phosphatase was decreased in those subjects treated with ABT-627, again demonstrating the effectivness of ABT-627 as an agent for treating prostate cancer.

### Chromagranin-A Levels (reference example)

The effect of ABT-627 adminstration on Chromagranin-A levels in human serum samples was determined by proprietary assay conducted at the Nichols Institute. The procedure is a two site chemiluminescence assay (ICMA) using one monoclonal antibody conjugated with biotin, another monoclonal antibody labeled with an acridinium ester, and an avidin-coated solid phase. The antibody/Chromagranin-A/antibody complex is bound to the solid phase by the avidin-biotin interaction and unbound materials are removed by washing. The bound, acridinium-labeled material produces light that is detected in a luminometer after addition of triggering agents. The Limit of Detection (LOD) for the assay was 0.07 ng/mL. As shown by the AUC in Figure 8, the average change from baseline for Chromagranin-A was higher for subjects treated with 2.5 mg/day of ABT-627, again demonstrating the effectivness of ABT-627 as an agent for treating prostate cancer.

### Interleukin-6 Levels (reference example)

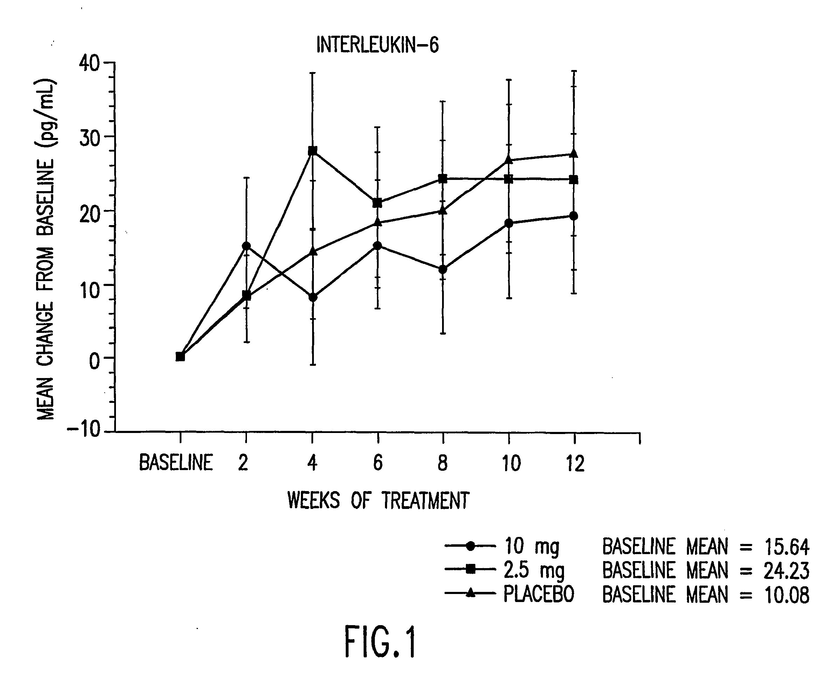

The effect of ABT-627 adminstration on Interleukin-6 levels in human serum samples was determined at Quintiles Laboratories using a sandwich immunoassay. Human serum samples and standards were incubated in microtiter plate wells coated with a monoclonal anti-IL-6 antibody, in the presence of a second monoclonal anti IL-6 antibody, linked to acetylcholinesterase. After incubation, the wells were washed, and the bound enzymatic activity was measured using a chromogenic substrate. The intensity of the color was proportional to the concentration of IL-6 in the sample or standard. As shown by the AUC Figure 1, the average change in baseline for Interleukin-6 was lower in those subjects treated with ABT-627, demonstrating the effectivness of ABT-627 as an agent for reducing inflammation and ameliorating pain.

### Bone Scan Methodology

Bone scans were performed with an NDA approved, Tc-99m phosphonate type radiopharmaceutical. This technique uses whole body format (skull to feet) so that anterior and posterior images are presented when using a 510 K-approved gamma camera. Alternatively, spot views covering both anterior and posterior projections of the total body can be obtained. Interpretation was performed according to standard nuclear medicine criteria, on a bone by bone basis, by recording the number of lesions at each site. Each site was evaluated against a confidence score of 1 to 5, where 1 is negative, 2 is probably negative, 3 is equivocal, 4 is probably positive, and 5 is definitely positive. The MSKCC (Clin. Can. Res. 1998; 4:1765-1772) was used to record these findings. For the purposes of scoring the extent of disease or the response to treatment, lesions with a confidence score of 4 and 5 were considered positive, and all other lesions were considered negative. In addition, in a blinded study, a reference nuclear medicine physician interpreted the bone scans quantitatively as follows: the percent of involved bone was estimated for each individual bone, and the individual bone involvement was summed to calculate a global percent bone scan index (BSI). More specifically, the bone scan was separated into three indices. The first was the appindicular scan which involved arms and legs (i.e. the humorous and all bones distal to the humerous and the femur and everything distal to the femur). The second was the axial (everything but the arms and the legs). The results of these scans were combined to provide the total BSI.

Bone scans were conducted on each subject on day one of the study, and on the final day of the study, and the changes from baseline in bone scan index scores were analysed by mean change and mean percent change, adjusting for baseline characteristics as co-variates using SAS version XXX software.

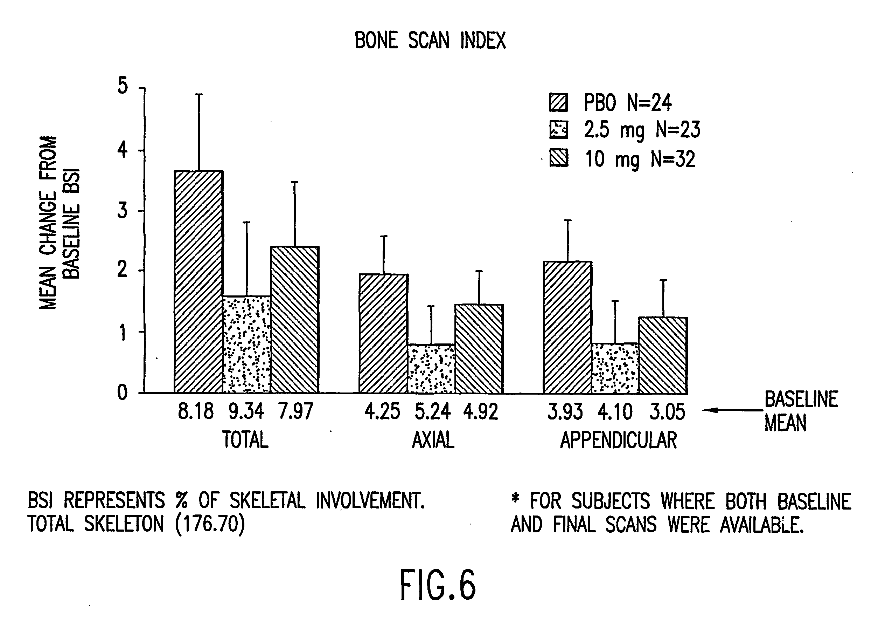

As shown in Figure 6, bone scans indicated a decrease in the proportion of total skeketal involvement in those subjects receiving ABT-627 versus placebo, demonstrating the effectivness of ABT-627 as an agent for reducing the fraction of total skeletal involvement by tumor.

### VAS Methodology/Administration/Analysis (reference example)

The Visual Analog Scale (VAS) is a common instrument of pain assessment performed by having a subject draw a vertical line on a 10 cm scale at the point that best describes his or her pain on average in the last 24 hours. A diagram of the scale is shown below:

| | |
|---|---|
| **No pain I--------------------------------I Pain as bad as it could** | |
| | possibly be |
| (not to scale) | |

During the course of the study, pain asessments were done daily, at bedtime, by the subject. If the subject was unable to maintain the log, a caregiver could complete the log on his or her behalf. The log also contained a table on which was recorded all daily pain medication consumed by the patient. The logs of daily VAS scores and analgesic consumption were collected at biweekly visits of the subject to the clinic when a new log was distributed. Clinical personnel who received the logs measured the score by measuring the distance (in mm) from the "no pain" end mark to the point where the subject's line crossed the VAS line. The number was written into the case report form next to the date the subject completed that page of the logbook.

Subjects with pain were initially stabilized in their pain so that their pain was treated to a tolerable and constant level. For this study, "tolerable and constant" refers to a pain score less than or equal to 5 cm on the VAS for an average of seven successive days while using four or less rescue doses of pain medication per day. A rescue medication dose refers to a dose equal to one single dose a patient used for common timed pain relief.

The weekly VAS scores were calculated excluding the lowest and highest score for each week and averaging the remaining five scores. If there were two days with the same VAS score, the day with the highest analgesic use was discarded.

The weekly mean VAS score was used to define subjects as responders or non-responders. A subject was considered a responder based on the reduction in the pain intensity: a weekly VAS score reduction of greater than or equal to 25% during at least two consecutive weeks without an increase of analgesic use during the same period (compared to baseline). Alternatively, a subject was considered a responder if his pain analgesic consumption was reduced by at least 25% during at least two consecutive weeks without a concomitant increase in VAS score.

The percentage of responders in each treatment group was compared to evaluate drug efficacy. The comparison was subjected to an adjustment for baseline characteristics and prognostic factors as co-variates, and the analysis was performed using the Cochran-Mantel-Haenszel test or a generalized linear model.

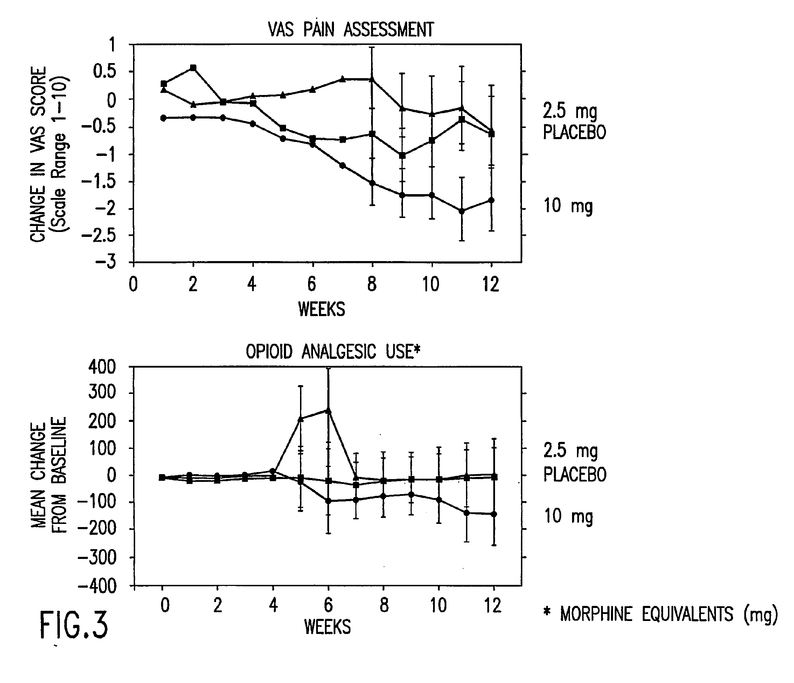

Weekly VAS scores are examined using a longitudinal analysis method to explore trends over time. The duration of the response, defined as the time from baseline to the last weekly assessment for which the responder definition was satisfied, was analyzed using the Kaplan-Meier methodology and logrank test. Cox proportional hazard models were used as needed (see U.S. Department of Health and Human Services. Management of Cancer Pain Clinical Practice Guidelines. AHCPR Publication #94-0592, Rockville, MD (1994). As shown by the AUC in Figure 3, VAS scores showed a decrease in pain, independent of the effects of morphine, after treatment with with ABT-627, demonstrating the effectivness of ABT-627 as an agent for ameliorating pain.

### Osteoblastic Activity and Bone Markers

Markers of osteoblastic activity were assessed using urine samples. Bone markers include bone alkaline phosphatase (BAP), deoxypridinoline, and N-telopeptide of Type I collagen. Blood samples were collected prior to dosing on Day 1, Day 42, Day 84, Day 168, and every 28 days after Day 168, with a final collection on the last day of the study.

### Bone Alkaline Phosphatase

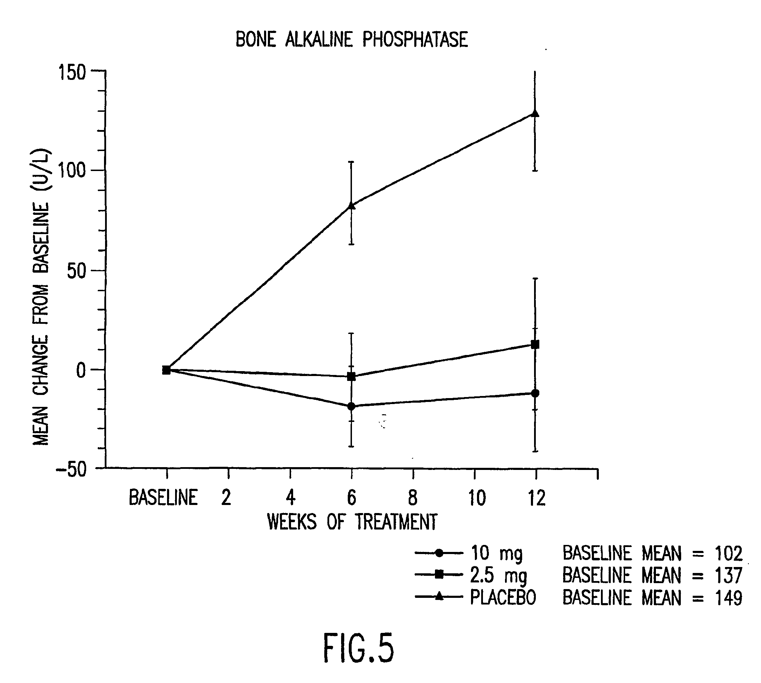

Bone Alkaline Phosphatase levels were determined using the bone-specific Alkphase-B^{®} assay published by Metra Biosystems (Mountain View, CA). As shown by the AUC in Figure 5, BAP levels decreased in subjects treated with ABT-627, demonstrating the effectivness of ABT-627 as an agent for inhibiting abnormal bone remodeling.

### Crosslinked N-Telopeptide Levels:

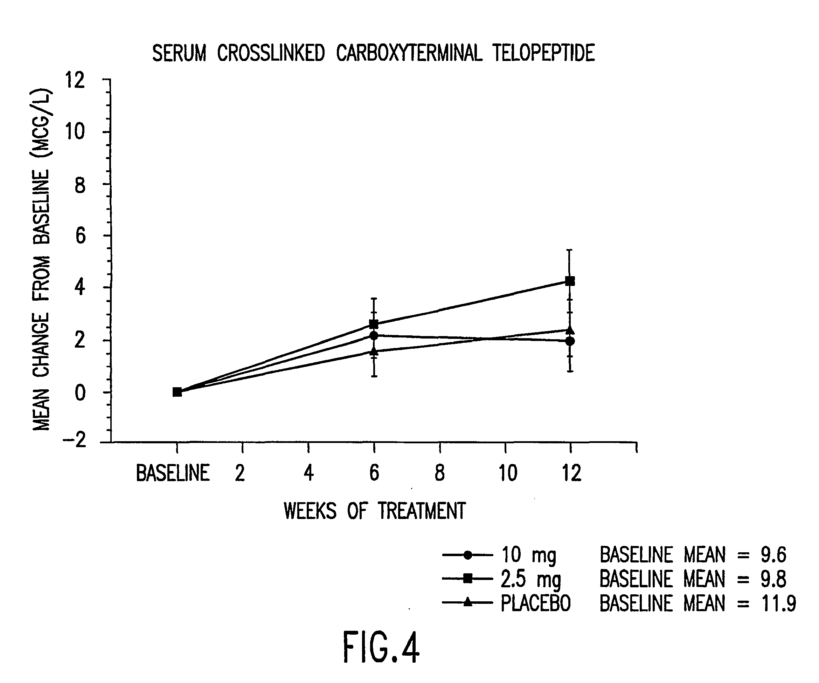

Cross-linked N-telopeptide levels were determined using the DiaSorin (Stillwater, MN) assay for the quantitative determination of carboxyterminal cross-linked telopeptide of type I collagen (ICTP) in human serum by equilibrium radioimmunoassay (RIA). Briefly, samples were incubated with the ¹²⁵I ICTP tracer and ICTP primary antibody for 2 hours at 37 °C. Following the 2 hour incubation, a pre-precipitated second antibody complex was added to separate the bound from free tracer. The assay was then centrifuged and decanted after a 30 minute incubation at room temperature. The bound tracer in the pellet was counted with a gamma counter. Counts were inversely proportional to the amount of ICTP present in each sample. As shown by the AUC in Figure 4, Crosslinked N-telopeptide levels decreased in subjects treated with ABT-627, demonstrating the effectivness of ABT-627 as an agent for inhibiting the bone remodeling associated with bone diseases.

## Claims

1. A compound of formula III: 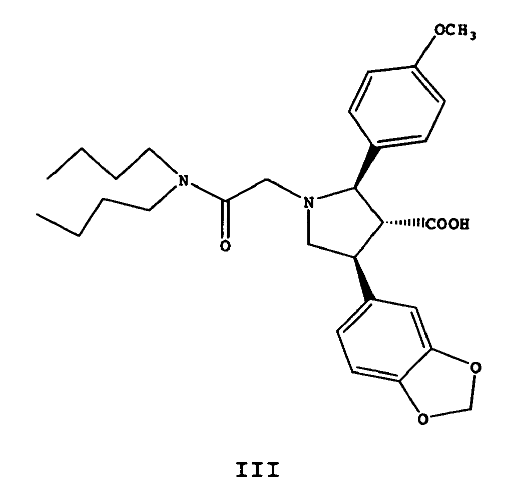 or a pharmaceutically acceptable salt thereof for inhibiting bone metastases in a man having prostate cancer by oral administration of a therapeutically effective amount to the man in need thereof.

2. The compound according to Claim 1 wherein the bone metastases are osteoblastic.

3. The compound according to Claim 2 wherein said administration is carried out additionally with the administration of an anticancer drug.

4. The compound according to Claim 2 wherein said administration is carried out additionally with the administration of radiation therapy.

5. The compound according to Claim 2 wherein said administration is carried out additionally with the administration of at least one therapeutic agent which impedes net bone loss.

6. The compound according to Claim 5 wherein the agent is a bisphosphonate.

## Patentansprüche

1. Eine Verbindung mit der Formel III 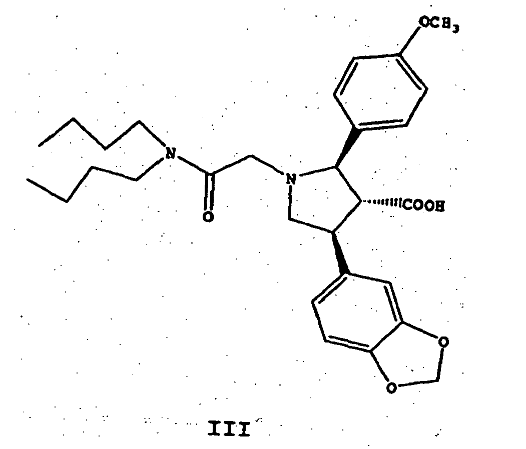 oder ein pharmazeutisch verträgliches Salz davon, zur Hemmung von Knochenmetastasen in einem Menschen, der Prostatakrebs hat, durch orale Verabreichung einer therapeutisch wirksamen Menge an den Menschen, der dies benötigt.

2. Die Verbindung gemäß Anspruch 1, worin die Knochenmetastasen osteoblast-isch sind.

3. Die Verbindung gemäß Anspruch 2, worin die Verabreichung zusätzlich zu der Verabreichung eines Antikrebs-Arzneimittels erfolgt.

4. Die Verbindung gemäß Anspruch 2, worin die Verabreichung zusätzlich zu der Verabreichung einer Bestrahlungstherapie erfolgt.

5. Die Verbindung gemäß Anspruch 2, worin die Verabreichung zusätzlich zu der Verabreichung von mindestens einem therapeutischen Wirkstoff, welcher den netto-Knochenschwund

6. Die Verbindung gemäß Anspruch 5, worin der Wirkstoff ein Bisphosphonat ist.

## Revendications

1. Composé de formule III : 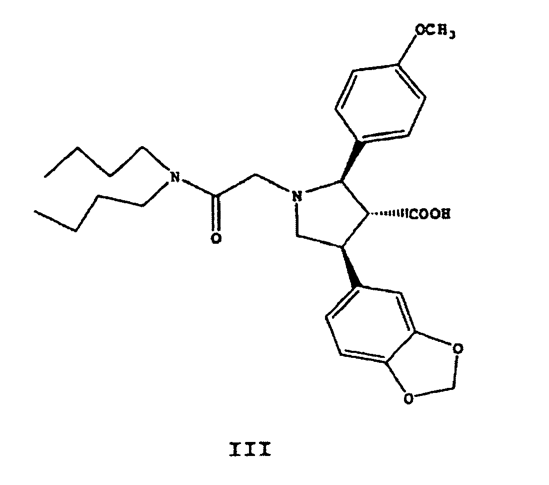 ou sel pharmaceutiquement acceptable de celui-ci pour inhiber des métastases osseuses chez un homme souffrant d'un cancer de la prostate par l'administration orale d'une quantité thérapeutiquement efficace à l'homme qui le nécessite.

2. Composé selon la revendication 1, dans lequel les métastases osseuses sont ostéoblastiques.

3. Composé selon la revendication 2, dans lequel ladite administration est réalisée en plus de l'administration d'un médicament contre le cancer.

4. Composé selon la revendication 2, dans lequel ladite administration est réalisée en plus de l'administration d'une radiothérapie.

5. Composé selon la revendication 2, dans lequel ladite administration est réalisée en plus de l'administration d'au moins un agent thérapeutique qui ralentit la perte osseuse nette.

6. Composé selon la revendication 5, dans lequel l'agent est un bisphosphonate.
